# EUROPEAN PATENT APPLICATION

(11) **EP 1 746 408 A1**
(43) Date of publication of application: **24.01.2007**
(21) Application number: 05106722.1
(22) Date of filing: 21.07.2005
(51) Int. Cl.: G01N 17/00

(54) **Improved apparatus and method for testing materials**

(71) Applicant: Electrolux Home Products Corporation N.V., 1930 Zaventem (BE)
(72) Inventor: Krupicka, Andreas, 33170, Pordenone (IT); Breese, Kevin, 33080, Roveredo in Piano (Pordenone) (IT)
(74) Representative: Giugni, Valter

(57) **Abstract**

Apparatus for testing the surface resistance properties of materials, comprising a parallelepiped casing, a pair of side runners extending horizontally parallel to each other at the same height from the bottom of said casing, a planar plate adapted to be associated to said pair of side runners, a hollow rotating spray arm adapted to rotate about a vertical axis and arranged within said casing in a position located below said plate, said rotating spray arm being provided with a plurality of perforations arranged on the upper side thereof and directed upwards, said spray means further comprising a pump adapted to convey an amount of liquid or semi-liquid substance through said rotating spray arm and said perforations, the latter being in turn so oriented as to cause said liquid substance, when pumped therethrough, to impart a rotary motion about said vertical axis to said rotating spray arm.

Said planar plate is provided with retaining means adapted to support, on the lower surface thereof, respective portions of the material to be tested.

## Description

The present invention refers to an improved kind of apparatus for testing surface characteristics and properties of materials, or the properties of surface treatments on materials, such as paint coatings, electroplated coatings and similar coatings deposited by any suitable process for protective and aesthetical purposes. It further relates to a testing method using this apparatus.

Widely known in the art is the need for surface properties, such as in particular the resistance of both materials and related surface coatings to be increasingly improved and, more generally, materials and related surface coatings of the most varied kinds to be suitably characterized in their behaviour against external agents of different natures that are known or likely to act in the long run upon the same materials and surface coatings thereof to eventually deteriorate or, in anyway, alter their surface properties.

Among these properties, wear resistance is certainly the most critical one to determine in general purpose and common products.

The reason for this is threefold. In the first place, it certainly does not take any sophisticated test equipment or engineering skill to detect what happens on the surface of a domestic product and possibly notice that it is being spoiled or damaged. In fact, any consumer can see this and thus rate the quality of the product simply on the basis of how the surface thereof actually withstands service life. In the second place, wear behaviour constitutes one of the most difficult properties to measure, since the actual contact mechanism between bodies highly depends on the actual operating conditions. Hence, only a well-selected set of test parameters can describe the actual wear behaviour and performance for a certain application. Finally, once a test method corresponding to real operating conditions has been eventually identified, quantitative evaluation still involves a problem in most cases. In general, it is desirable to find a way to quantitatively rate wear behaviour and performance in agreement with visual impression. The reason for this is quite simple: as far as domestic appliances are concerned, in the majority of cases the critical aspect of wear is not the resulting reduction in functionality, but rather the detrimental effects on appearance, i.e. the loss of aesthetical appeal.

In the past, the common approach has been based on following the procedures dictated mainly by coating producers and the automotive industry in general, which have relied to a great extent on relatively simple standard tests. However, as cases of discrepancies and poor agreement between predicted wear performance in standard test procedures and actual wear performance in several practical applications have kept being reported to an increasing extent throughout industry in these last few years, an increasing number of companies have started developing their own test procedures to more effectively cope with actual operating conditions. In general, these tests might be referred to as simulative tests, since they simulate a certain operating condition. Meanwhile, raw material producers and coating suppliers have started to show an increasing interest in more sophisticated instrumental apparatus and methods that would contribute to a better understanding of hardness, scratch resistance and wear. These tests might be referred to as laboratory-based inferential tests, since both contact conditions and residual deformations are accurately controlled and, thus, more easily linked with parameters that relate to inherent properties such as stiffness, hardness, elastic recovery and toughness. The major drawbacks that are generally encountered with these two approaches are a limited correlation to more than a single wear condition and the higher costs involved as compared with the existing standard methods, respectively.

In recent years, a lot of effort has been made in view of establishing improved, more effective methods for assessing and evaluating wear performance, as well as finding at least a specific test procedure for each application. However, the general demands for good correlation capability and a reasonable price have constituted major obstacles and resulted in limited success. In the light of the strong demand for the development of improved abrasion test methods in all industry and business sectors working with or using materials (from raw material manufactures down to end-users), there have been issued several patents in the last decade covering both test equipment and methods allegedly aimed at enabling abrasion and wear to be measured in a more effective manner. However, none of these test methods succeeded in establishing itself to any satisfactory extent in the current practice, thereby failing to convince both appliance manufacturers in general and their suppliers of their applicability. There have been two main reasons for that, in the sense that they have either been providing just limited improvement over existing standard procedures or have simply proved much too expensive.

Various solutions have been identified and suggested in the course of the last few years in view of solving the above-mentioned problem of providing a testing apparatus, along with a related testing method, that would prove simple, effective, capable of ensuring reproducibility of both test conditions and test results and, above all, extremely low-cost and reliable.

Widely known in the prior art - as exemplified in this case by the Japanese Patent Application No. 2001010584 - is the practice of using a so-called saline test chamber, in which there is injected a mixture of test substances aimed at attacking and affecting - in a way to be found out or ascertained, the material to be tested that is appropriately placed inside the chamber. Such solution, although certainly effective and simple, calls anyway for the use of test equipment of a professional kind and, as a result, quite expensive. Moreover, a drawback that is generally connected with controlled-atmosphere test chambers of a traditional type lies in the fact that these chambers, while enabling accelerated tests to be carried out, do not however allow for also the mechanical effect to be identified and rated, which is due to the test substances thrown directly against the surface of the material to be tested impinging thereupon.

Even more, this method is limited to only metallic substrate as a consequence of being a test for evaluating corrosion resistance under accelerated conditions.

Known from the disclosure in WO 02/12880 is a test chamber, in which there is arranged a spray nozzle adapted to inject an abrasive powder that is thrown against the items to be tested; this spray nozzle, however, is only able to throw such abrasive powder from a single punctiform position, so that the test being performed is critically impaired in both its rapidity and effectiveness.

Disclosed in the Japanese patent application No. 59207995 is the practice of carrying out a tablet abrasion test by introducing these tablets inside a drum that is then caused to rotate; this requirement, i.e. the drum having to be driven rotatably, implies of course a corresponding operating and constructive complexity - due to the kinematical mechanisms and arrangements required - and makes the related apparatus much more expensive.

Known from the disclosure in the Japanese patent application No. 02238007 is the practice of using a chamber for submitting material samples to abrasion tests, in which this chamber is stationary, while the test samples are caused to rotate by appropriate kinematical arrangements. Similarly to the above-cited case, also this solution implies a constructive and operating complexity that is instrumental in increasing the related costs.

It would therefore be desirable, and is actually a main object of the present invention, to provide an apparatus for testing surface properties of materials, or treatments and/or coatings applied on these materials, which enables the effect of chemical or abrasive substances being thrown in a cyclically repeated manner for a great number of times against the same surface of said materials and/or coatings provided in a sensible number of samples thereof to be examined and identified. This test apparatus shall further be capable of doing away with all of the afore-cited drawbacks and, in particular, shall enable the test samples to be kept stationary, i.e. in a fixed position inside a stationary test chamber.

Furthermore, the nozzles provided to eject said chemical or abrasive substances shall be arranged in such position and provided in such number as to reliably enable a satisfactorily large specimen surface to be tested in a uniform manner; moreover, the pumping action of the chemical and/or abrasive test medium against the material to be tested, as well as and the cyclic occurrence and rate thereof, shall be obtained in an absolutely simple, reliable and inexpensive manner.

Such apparatus shall of course be capable of being manufactured using existing, readily available techniques and materials, and it shall be competitive in its construction and easy to use. It is further desirable that such apparatus be capable of being embodied by just introducing slight modifications in currently produced appliances, i.e. through the use of appliances out of the current production that have been slightly modified to perform as a testing apparatus of the above-cited kind.

According to the present invention, these aims are reached in a particular kind of apparatus for testing surface properties of materials, or the properties of surface treatments and/or coatings applied on these materials, some preferred, although not sole embodiments of which are described below by way of non-limiting example with reference to the accompanying drawings, in which:
- Figure 1 is a symbolical see-through front view of a test apparatus according to the present invention;

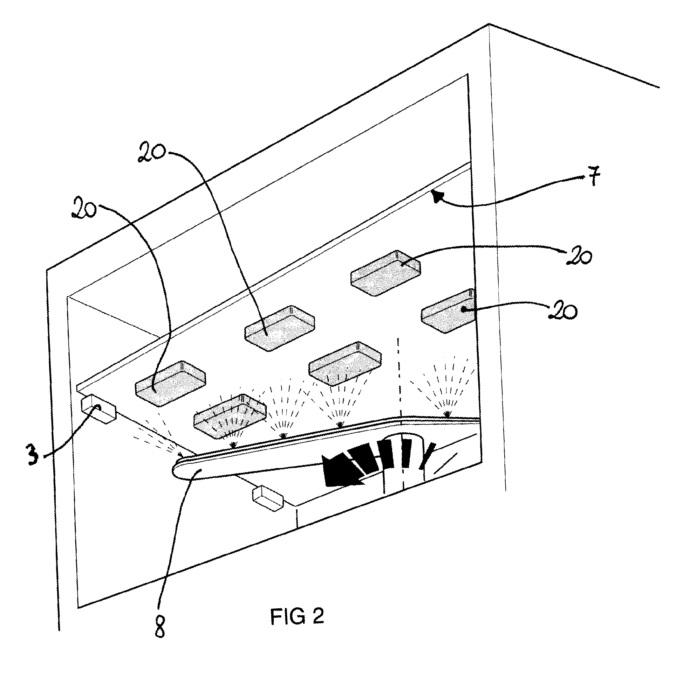

- Figure 2 is a perspective bottom view of the internal parts of a test apparatus according to the present invention;
- Figure 3 is a view of a different embodiment of the test apparatus shown in Figure 1;
- Figures 4 and 5 are two perspective views illustrating two respective details in the embodiment of a test apparatus according to the present invention;
- Figures 6 and 7 are views of respective embodiments of two devices in a test apparatus according to the present invention;
- Figure 8 is a view of a particular conformation of a part of a test apparatus according to the present invention.

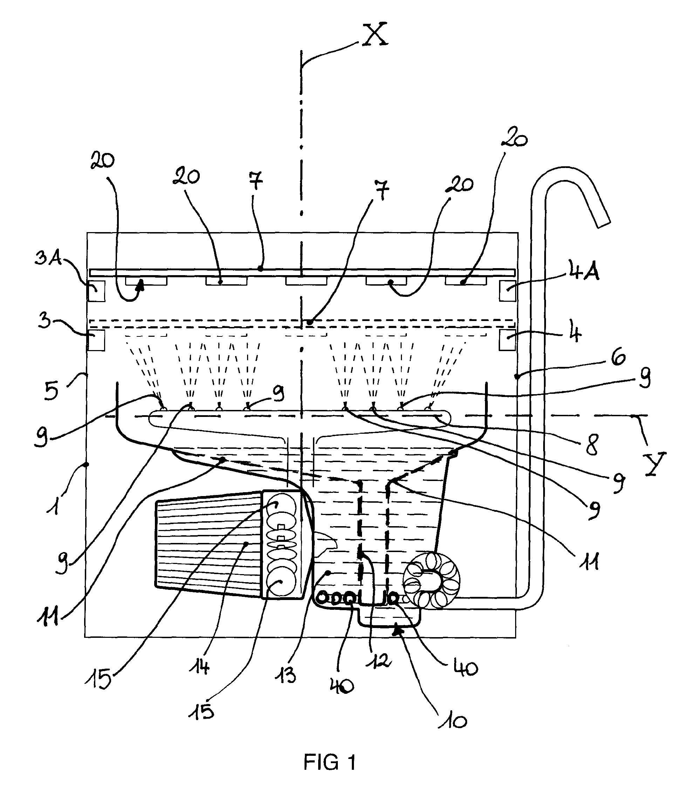

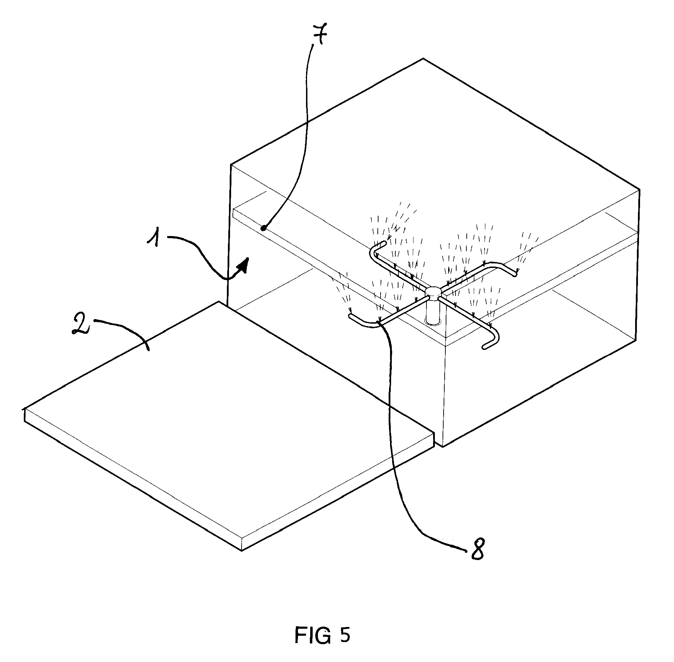

With reference to Figures 1 and 5, a test apparatus according to the present invention comprises:
- an enclosed outer casing 1 in the shape of a quadrilateral parallelepiped with vertical walls, in which the material to be tested is to be placed,
- an outer access door 2 opening in a vertical wall of said casing,
- at least a pair of side tracks or runners 3, 4 extending parallel to each other, and at the same height, along two mutually opposed side inner walls 5, 6 of said casing,
- a flat plate or board 7 provided in such a size and shape as to be able to be inserted in or laid upon said two side runners 3, 4 by passing through said access door 2,
- a hollow rotating spray arm 8 arranged on the bottom of said outer casing and provided with a plurality of perforations 9 that are directed upwards and, hence, towards the bottom surface of said flat plate.

The above-mentioned rotating spray arm is adapted to rotate about a vertical axis X that is substantially centered in the middle portion of said outer casing.

Into said rotating spray arm, and from the bottom thereof, there is let a stream of liquid substances, which shall be dealt with in greater detail further on in this description, and which - as appropriately pumped with the aid of generally known means - flow through said rotating spray arm to be eventually ejected upwards through said perforations, thereby being converted into a plurality of spray-like jets.

The above-cited perforations are so shaped and oriented - relative to the substantially horizontal axis Y of said rotating spray arm - as to ensure that the ejection of the respective jets from said rotating spray arm will cause a mechanical reaction to take place in the same rotating spray arm, which in turn causes the latter to rotate in a continuous manner. Furthermore, due to the effect of such rotation the jets themselves turn into jets that, while keeping constantly oriented upwards, move around, i.e. with a rotary motion, due to them having the respective origins that rotate along respective circles arranged on the horizontal plane formed by the rotation plane of the rotating spray arm. Owing to this, each jet will therefore hit periodically, i.e. at each rotation of the rotating spray arm, a circumscribed area on the bottom or lower surface of said flat plate and, ultimately, as it moves rotatorily in the above-described manner each single jet will hit a respective circular area on the lower surface of said flat plate.

It will at this point have been fully realized that described above there has been an apparatus, the general structure of which, along with the related mode of operation, is fully similar to the structure and way of operating of a household-type dishwashing machine of the kind that is readily available on the market at relatively moderate purchase prices.

It should right away be pointed out that the basic advantage of the present invention lies in the possibility for an already existing structure to be used in view of providing, through just some minor constructive and operating modifications thereof, a materials testing apparatus that is fully effective, efficient, highly flexible and convenient to use.

To this purpose, therefore, use is made basically of a household-type dishwashing machine that is provided with the afore-described means, can be either produced or modified at extremely reduced costs, and is since the beginning capable of substantially ensuring the required functionality.

Therefore, according to the preferred embodiment of the present invention, which is based on such use of the structure of a household-type dishwashing machine, the casing 1 may be obtained from the washing vessel, or tank, of the dishwashing machine from which the testing apparatus is being derived, while the hollow rotating spray arm 8 is readily obtainable in the same way in which the lower rotating spray arm of such dishwashing machine is designed and manufactured.

The flat plate 7 may be made with the use of conventional materials and techniques, and is so sized as to be able to be removably inserted in or laid upon or, anyway, associated with said side runners 3, 4.

Also these side runners can be made with the help of generally known means, fully similar to the ones used to make the side runners or tracks used to guide and sustain the dish-carrying racks that are inserted in the washing vessel of a dishwashing machine.

The flat plate 7 is therefore hit from the bottom by a plurality of jets of a liquid being ejected through the respective perforations or spouts; as a result, the samples or portions 20 of the materials to be tested can be subdivided into preferably planar specimens of a limited size to be applied onto the lower surface of the plate 7, so as to cause them to be exposed to one or more of said rotating and upward-directed jets of liquid hitting them.

As a result, when combined with the nature of the liquid being so sprayed, such mechanical action that is performed cyclically, but can be continued for even quite extended periods of time, by said jets of liquid hitting said specimens from the bottom brings about the desired accelerated test effect on the outer surface of the specimens of the material and/or coating to be tested.

The above-described apparatus is intentionally provided so that the specimens are hit from the bottom by jets being ejected from the lower rotating spray arm, i.e. the rotating spray arm located at the bottom of the apparatus. In fact, if the specimens were on the contrary due to be placed on the upper surface of the plate 7 so as to be hit by jets ejected from the top, this would unavoidably have the effect of causing material and substances in the liquid being ejected to keep dwelling on and pile up on the specimens themselves, thereby generating a kind of protecting effect that would alter both the contact/impingement action and - of course - the final result of the test.

As for the rest, the test apparatus replicates the structure and the functionality, i.e. mode of operation, of a conventional household-type dishwashing machine: on the bottom 10 of the casing 1 there is arranged a funnel 11 for collecting the ejected test liquid, which is conveyed by gravity into a sump 13.

However, in case of using test liquid mixed with sand, the filter has to be removed, that is one of the important modifications of a dishwasher; this because the particle flow has to be maintained constant when the machine is operated in a continuous "non-stop" mode.

From this sump 13, a pump 14 - generally known as such in the art - sends a stream of liquid collected in said sump into said hollow rotating spray arm 8 via at least a conduit 9 firmly joined with said pump.

As far as the test liquid itself is concerned, it can be of any suitable kind, or consists of any suitable mixture, provided of course that the parts of the test apparatus that are due to come into contact with said liquid are adequately resistant thereto. Particularly effective for abrasion tests is a kind of mixture formed by a mass of water in which there is dispersed an appropriate amount of abrasive material, such as very fine sand (e.g. Fontainbleau sand).

Use can of course be made of the most varied kinds and types of chemical agents, such as etching agents, acids, bases, and the like, as far as the apparatus itself is adequately protected against them, i.e. will not be affected by them.

Particularly advantageous is the fact that the sole impeller vanes 15 of the pump 14 are made of metal, such as in particular stainless steel, rather than plastic, since it is actually just these vanes that are exposed to the strongest mechanical action of the test liquid.

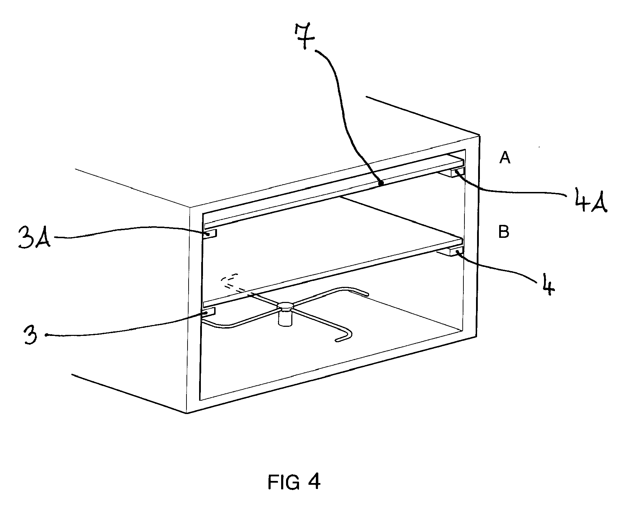

With reference to Figures 1 and 4, an advantageous improvement consists in providing the casing not with a single pair of side runners 3, 4, but rather with a plurality of such pairs 3A, 4A, 3B, 4B, etc., in which each such pair of runners is arranged at a different height. This practically enables the distance of the specimens from the rotating spray arm on the bottom to be adjusted in a quite easy and convenient manner by simply selecting the proper pair of side runners, which the flat plate 7 is to be associated with. This ultimately enables the force to be controlled, with which the liquid ejected from the related perforations or spouts hit said specimens from the bottom.

In Figure 4, obviously the flat plate 7 can assume only one position at a time, i.e. engaging the runners 3,4 or 3A, 4A.

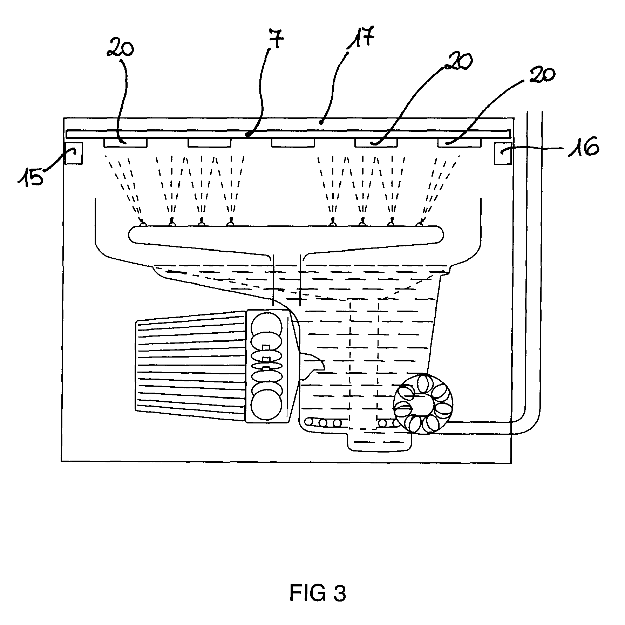

With reference to Figure 3, in view of keeping the overall height of the apparatus as low as possible, as well as making use of more inexpensive structures, the height of the casing is reduced to a lowest acceptable value, while the pair of parallel side runners 15, 16 are located immediately below the top inner wall 17 of said casing 1.

As regards the way in which the portions or specimens 20 of the materials to be tested can be applied onto the lower surface of said plate 7, a number of simple means and methods, as generally known as such in the art, can be actually used to such purpose. Anyway, although a most simple and readily done method in this connection consists in having the specimens bonded to such surface by means of an adhesive in a normal manner, such solution has however a major drawback in that, when the specimens shall eventually be removed from said surface, e.g. at the end of the test, this may prove quite difficult and awkward to perform, mainly due to the risk of damaging either the specimens themselves or the plate supporting them. This difficulty can be overcome by using melt glue, a common inexpensive easy-to-use type of adhesive that is tough but very elastic and not stiff making it easy to remove samples without damage using a sharp blade or similar. However in the long run the progressive overlapping of glue layers may prevent a firm and reliable sample sticking.

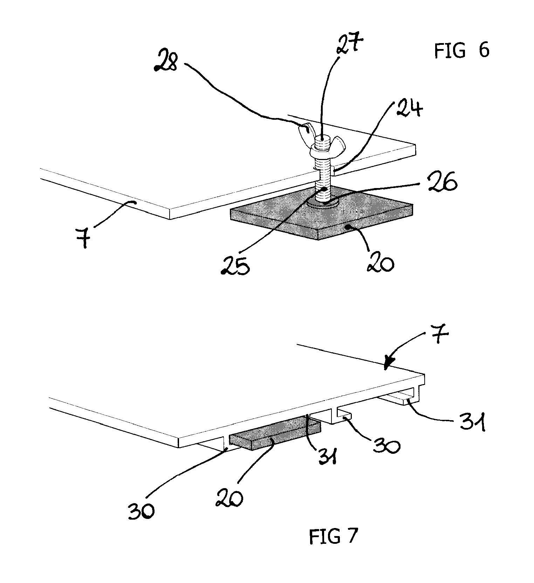

For such drawbacks to be effectively avoided, the solution is advantageously adopted consisting in providing attachment or retaining devices that are adapted to enable said specimens 20 to be selectively attached to and released from the support plate 7 without any use of adhesives and, hence, without any adhesive bond having to be undone or torn apart. With particular reference to Figure 6, these retaining devices are constituted by a plurality of through-bores 24 extending through said plate 7, and a corresponding plurality of through-passing means, such as for instance rods or bolts 25, which are inserted in the respective through-bores 24 and are adapted to be coupled with the lower end portion thereof 26 to the upper surfaces of respective specimens 29, while the upper end portion 27 thereof is clamped, e.g. with the use of a wing nut or lock-nut 28, or similar non-destructive locking means, so that said rods or bolts 25 cannot slip off the respective through-bores 24.

With reference to Figure 7, an alternative embodiment may simply consist in providing the lower surface of said plate 7 with one or more pairs of grooves, ribs or similar guide means 30, 31, which are so protruding and shaped as to form corresponding accommodations in which said specimens 20 can be easily and conveniently inserted and removed by causing them to slide in and off horizontally.

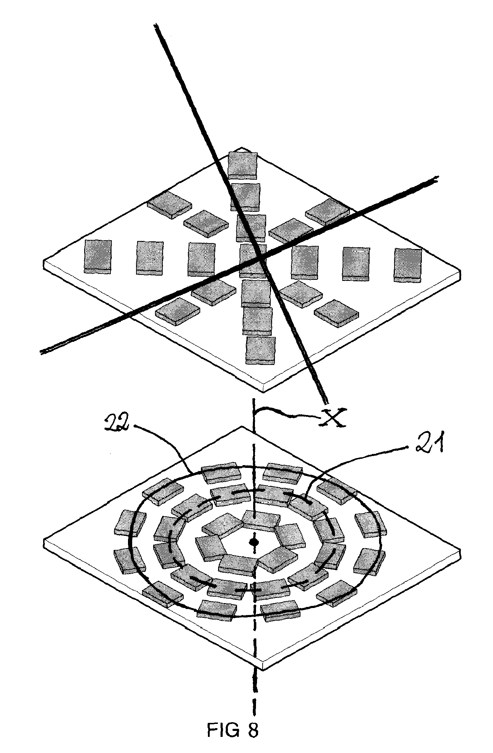

With reference to Figure 8, said retaining or support means, regardless of them being provided in the form of bolts passing through corresponding bores in the plate or in the form of pairs of grooves or ribs as described above, or in any other form, are advantageously arranged according to a pattern of concentric circles 21, 22, etc., whose centre is the intersection of said vertical axis X of rotation of the rotating spray arm 8 with said planar plate 7. The advantage of such solution lies in the fact that, if the radii of said concentric circles 21, 22 are equal to the distances of said perforations 9 from the vertical axis X of rotation of the rotating spray arm 8, then the jet of liquid issuing from each such perforation 9 will hit in a direct manner just those specimens that are located along the same circle having a radius that is equal to the distance of the corresponding perforation 9 from said axis X. It will be readily appreciated that this condition is instrumental in enhancing the efficiency of the test, since it enables the number of specimens that can be directly hit by the jets of liquid to be optimised accordingly.

More over, the advantage depends not only on the increased number of test specimens, but also on the fact that each radial position correspond to different test conditions in terms of spray pressure/intensity, as sample on the inner line can be subjected to a wear that, according to the lab test, is much higher than the wear on the samples on outer line. The wear rate depends for the present spray arm on radial position, constant comparable wear can only be obtained for a certain radius to centre. Hence another advantage is the variation of wear rate: one can run tests that correspond to different wear rate at the same time.

Finally, in view of increasing the variety of tests that can be carried out, e.g. by including also the effect of temperature among the test parameters, on the bottom 10 of the casing 1 there may be advantageously installed a heating element 40, preferably of the sheathed type for submerged operation as widely known as such in the art, which is adapted to heat up in a controllable manner, implemented e.g. by a controlled loop between this heater and some thermocouples located close to the tested samples, the mass of liquid substance being used to test purposes. The selection of the type of heating element to be used, the rating thereof, as well as its installation and the manner and means for controlling it, are fully within the ability of those skilled in the art, so that they shall not be described here any further for reasons of brevity.

## Claims

1. Apparatus for testing the surface resistance properties of materials, comprising:
- a casing in which the materials to be tested are placed,
- support means, onto which said materials to be tested are applied,
- spray means adapted to eject and convey a pre-defined amount of abrasive or chemically active substances against said materials to be tested,
**characterized in that**:
- said casing is a substantially parallelepiped casing (1) adapted to be closed by a vertical door (2) on a side thereof, and provided with an outward opening door on a vertical wall thereof,
- said support means include:
a) a pair of side runners (3, 4 - 3a, 4a) extending horizontally at the same height from the bottom of said casing, and extending parallel to each other along two respective opposite vertical walls (4, 6) of said casing (1),
b) at least a substantially planar plate (7) having a shape substantially corresponding to the inner cross-sectional shape of said casing, and being so sized as to enable it to be slidably inserted in said pair of side runners,
- said spray means comprise at least a hollow rotating spray arm (8) adapted to rotate about a vertical axis (X) and arranged within said casing in a position located below the horizontal plane defined by said pair of side runners, said rotating spray arm being provided with a plurality of perforations (9) arranged on the upper side thereof and directed upwards, said spray means further comprising hydraulic pumping means (14) adapted to convey an amount of liquid or semi-liquid substance through said rotating spray arm (8) and said perforations (9).

2. Apparatus for testing materials according to claim 1, **characterized in that** said perforations (9) are so oriented relative to the axis (Y) of said rotating spray arm (8) as to cause said liquid substance, when pumped therethrough, to impart a rotary motion about said vertical axis (X) to said rotating spray arm.

3. Apparatus for testing materials according to claim 2, **characterized in that** said support means include a plurality of pairs of side runners (3A, 4A), in which the runners of each such pair are arranged horizontally and applied at the same height relative to the bottom of said casing, and extend parallel to each other along two respective opposite vertical walls (5, 6) of said casing, the runners of different pairs being arranged on respective planes at differentiated heights.

4. Apparatus for testing materials according to claim 2 or 3, **characterized in that** said planar plate (7) is provided with retaining means adapted to support, on the lower surface thereof, respective portions (20) of the material to be tested.

5. Apparatus for testing materials according to claim 4, **characterized in that** said retaining means are arranged according to concentric circles (21, 22, ...) around the vertical axis (X) of rotation of said rotating spray arm.

6. Apparatus for testing materials according to claim 4, **characterized in that** said retaining means comprise respective through-bores (24) extending through said planar plate (7).

7. Apparatus for testing materials according to claim 4, **characterized in that** said retaining means comprise respective pairs of grove and/or ribs and/or runners (30, 31) applied to and/or arranged on the lower surface of said planar plate (7).

8. Apparatus for testing materials according to any of the preceding claims, **characterized in that** said pumping means comprise a pump (14), the impeller vanes (15) of which are made of metal, preferably stainless steel.

9. Apparatus for testing materials according to any of the preceding claims, **characterized in that** said pumping means are adapted to suck in said liquid substances from the bottom (10) of said casing (1) and pump them into said rotating spray arm (8).

10. Apparatus for testing materials according to any of the preceding claims, **characterized in that** the side runners (15, 16) of the pair of runners arranged in the highest position, are positioned immediately below the inner surface of the upper wall (17) of said casing.

11. Apparatus for testing materials according to any of the preceding claims, **characterized in that** it is also provided with heating means (40) for selectively heat up said liquid or semi-liquid substances.

12. Method for carrying out accelerated tests of the surface resistance properties of materials using the apparatus according to any of the preceding claims, **characterized in that** it comprises the steps of applying specimens of the material to be tested on to the lower surface of a support plate, inserting said support plate in respective pairs of runners inside an enclosed casing, and spraying for a continuous, pre-set length of time a plurality of jets of a liquid substance against said specimens, the ejection spouts of which are oriented upwards and are arranged on a rotating device in a position situated below said support plate, around a vertical axis (X) that is substantially orthogonal to said support plate.
